# EUROPÄISCHE PATENTSCHRIFT

(11) **EP 2 179 822 B1**
(45) Veröffentlichungstag und Bekanntmachung des Hinweises auf die Patenterteilung: **19.01.2011**
(21) Anmeldenummer: 09013412.3
(22) Anmeldetag: 23.10.2009
(51) Int. Cl.: B25J 9/00, A61B 6/04

(54) **Halterungsvorrichtung, medizinischer Roboter und Verfahren zum Einstellen des Tool Center Points eines medizinischen Roboters**
Holder device, medical robot and method for adjusting the tool centre points of a medical robot
Dispositif de fixation, robot médical et procédé de réglage du point central d'outil (TCP) d'un robot médical

(30) Priorität: 24.10.2008 DE 102008043156
(43) Veröffentlichungstag der Anmeldung: 28.04.2010
(73) Patentinhaber: KUKA Roboter GmbH, 86165 Augsburg (DE)
(72) Erfinder: Jacob, Dirk, 87616 Marktoberdorf (DE)
(74) Vertreter: Funk, Alexander Tobias

(56) Entgegenhaltungen:
- EP-A2- 1 529 605
- WO-A1-98/04194
- WO-A1-98/17177
- DE-A1-102005 041 606
- US-A- 4 400 820
- US-A- 5 971 997
- US-A1- 2008 317 216

## Beschreibung

Die Erfindung betrifft eine Halterungsvorrichtung, einen medizinischen Roboter und ein Verfahren zum Einstellen des Tool Center Points eines medizinischen Roboters.

Roboter im Allgemeinen sind Arbeitsmaschinen, die zur automatischen Handhabung und/ oder Bearbeitung von Objekten mit Werkzeugen ausgerüstet werden können und in mehreren Bewegungsachsen beispielsweise hinsichtlich Orientierung, Position und Arbeitsablauf programmierbar sind. Roboter weisen üblicherweise programmierbare Steuerungen (Steuerungsvorrichtungen) auf, die während des Betriebs die Bewegungsabläufe des Roboters steuern.

Roboter werden außerdem zunehmend in der Medizintechnik z.B. als Träger von Patientenlagerungssystemen eingesetzt. Beispielsweise die DE 10 2005 041 606 A1 offenbart eine Patientenpositioniervorrichtung zum Positionieren eines Patienten in einer Bestrahlungsposition für eine Strahlentherapieanlage. Die Patientenpositioniervorrichtung umfasst ein Patientenhalterungsmodul und einen das Patientenhalterungsmodul bewegenden Positionierarm, dessen Bewegung ein Therapiekontrollzentrum steuert.

Die US 4,400,820 offenbart eine Kopffixiervorrichtung, die Öffnung umfasst, die mit den Hörkanälen des mittels der Fixiervorrichtung fixierten Kopfes fluchten.

Im Bereich der Hals-Nasen-Ohren Medizin existieren unterschiedliche den Gleichgewichtssinn betreffende Krankheiten. Ein Beispiel einer solchen Krankheit ist der benigne paroxysmale Lagerungsschwindel (BPLS), bei der betroffene Personen über Drehschwindel klagen, sobald sie die Lage ihrer Köpfe ändern. Ausgelöst wird dieser Lagerungsschwindel durch ein Ablösen von Otolithenpartikel der macula utriculi, die dann in den Bogengängen des betroffenen Ohres eine Reizung der Nervenzellen verursacht, die wiederum den Schwindel verursacht.

Die Beschwerden können über eine gezielte Positionierung der betroffnen Personen, wie z.B. von John M. Epley beschrieben, therapiert werden. Durch eine gezielte und geometrische Abfolge von Umlagerungen der betroffenen Person lösen sich die Partikel ab und werden in einen Bereich des Vestibulum des vestibulären Labyrinths geschwemmt, wo sie keine Beschwerden mehr verursachen.

Abhängig von den betroffenen Bogengängen wird die betroffene Person zum Teil mittels komplexen Abläufen umgelagert. Insbesondere sind die dem vertikalen und dem hinteren Bogengang zugeordneten Umlagerungen kaum oder gar nicht durchführbar. Außerdem bedarf es zum Teil einer relativ genauen Lagerung der betroffenen Person und der zeitliche Ablauf des Umlagerns ist ebenfalls relativ kritisch. Um einen relativ guten therapeutischen Erfolg zu erzielen, sollte die Bewegung der betroffenen Person bezüglich des Zentrums des betroffenen Vestibularorgans (Gleichgewichtsorgans) erfolgen.

Aufgabe der Erfindung ist es, eine Vorrichtung anzugeben, die Vorraussetzungen für einen verbesserten Erfolg bei der Behandlung des oben erwähnten Lagerungsschwindels erlaubt.

Die Aufgabe der Erfindung wird gelöst durch eine Halterungsvorrichtung zum Halten einer Person, aufweisend eine Fixiervorrichtung zum Fixieren des Kopfs einer von der Halterungsvorrichtung gehaltenen Person, wobei die Fixiervorrichtung derart ausgeführt ist, dass sie aufgrund ihrer dem Kopf der Person zugeordneten Einstellung einen Rückschluss auf die Position der Öffnungen der Gehörkanäle der Ohren der Person relativ zur Halterungsvorrichtung erlaubt, wobei die Fixiervorrichtung Messvorrichtungen aufweist, deren Signale einen Rückschluss auf die Position der Öffnungen der Gehörkanäle der Ohren der Person erlauben.

Ein weiterer Aspekt der Erfindung betrifft einen medizinischen Roboter, aufweisend
- die erfindungsgemäße Halterungsvorrichtung,
- eine Steuerungsvorrichtung und
- einen Roboterarm, der von der Steuerungsvorrichtung angesteuerte mehrere Achsen und eine Befestigungsvorrichtung aufweist, wobei die Halterungsvorrichtung an der Befestigungsvorrichtung befestigt ist.

Ein weiterer Aspekt der Erfindung betrifft ein Verfahren zum Einstellen des Tool Center Points eines Roboters, der einen Roboterarm mit einer Befestigungsvorrichtung aufweist, an der eine Halterungsvorrichtung mit einer Fixiervorrichtung befestigt ist, die insbesondere die erfindungsgemäße Halterungsvorrichtung ist, aufweisend folgende Verfahrensschritte:
- Fixieren des Kopfes einer mit der Halterungsvorrichtung gehaltenen Person mittels der Fixiervorrichtung,
- Ermitteln der Position eines der beiden Trommelfelle der Person mittels Messvorrichtungen der Fixiervorrichtung relativ zur Halterungsvorrichtung,
- Ermitteln der Position des dem Trommelfell zugeordneten Gehörorgans relativ zur Halterungsvorrichtung und
- Legen des Tool Center Points des Roboters in die Position des Gehörorgans.

Die erfindungsgemäße Halterungsvorrichtung, die z.B. als eine Patientenliege ausgebildet ist, umfasst die Fixiervorrichtung, mit der der Kopf der Person relativ zur Halterungsvorrichtung fixierbar ist. Die Fixiervorrichtung ist derart ausgebildet, so dass aufgrund ihrer der Person zugeordneten Einstellung einen Rückschluss auf die Positionen der Öffnungen der Gehörkanäle relativ zur Halterungsvorrichtung erlaubt. Dies kann z.B. dadurch erreicht werden, indem die Fixiervorrichtung geeignete automatische Messvorrichtungen umfasst, mittels derer die Öffnungen der Ohren der Person, sobald deren Kopf mit der Fixiervorrichtung fixiert ist, umfasst. Automatische Messvorrichtungen umfassen z.B. Positionsmessvorrichtungen. Es ist aber auch möglich, dass aufgrund der mechanischen Einstellung der Fixiervorrichtung z.B. eine Bedienperson die Position der Öffnungen der Ohren mechanisch an der Fixiervorrichtung ablesen kann.

Ist die Position der Öffnung des Ohres relativ zur Halterungsvorrichtung bekannt, dessen Gehörorgan den Schwindel auslöst, dann kann aufgrund dieser Information auf die Position des relevanten Gehörgangs rückgeschlossen werden, um z.B. den erfindungsgemäßen Roboter derart anzusteuern, so dass dieser eine für die Therapie der Person vorgesehen Bewegung bezüglich der Position des relevanten Gehörorgans ausführen kann.

Der erfindungsgemäße Roboter kann dann eingerichtet sein, aufgrund der mittels der Fixiervorrichtung ermittelten Position einer der Öffnungen der Gehörkanäle der Person den Tool Center Point, dessen Bewegung im Raum mittels der Steuerungsvorrichtung steuerbar ist, in Relation zu dieser Öffnung, insbesondere in die Position des relevanten Gleichgewichtsorgans einzustellen.

Die Fixiervorrichtung kann eine Messvorrichtung aufweisen, die bei der dem Kopf der Person zugeordneten Einstellung an den Öffnungen der Gehörkanäle anliegt und die Entfernung zu den Trommelfellen der Person ermittelt. Gehörkanäle verschiedener Personen sind verschieden lang. Aufgrund dieser Ausführung der Fixiervorrichtung ergeben sich Vorraussetzungen für eine verbesserte Bestimmung der Position des dem zu behandelnden Gehörorgans zugeordneten Trommelfells, wodurch ebenfalls die Position des relevanten Gehörorgans relativ zur Halterungsvorrichtung und somit gegebenenfalls auch zum Roboter genauer bestimmt werden kann. Die Messvorrichtung basiert bevorzugt auf berührungslosen Messverfahren, wie z.B. Laser oder Ultraschall. Es können aber auch kontaktbehaftete Messvorrichtungen verwendet werden.

Der erfindungsgemäße Roboter kann dann eingerichtet sein, aufgrund der mittels der Fixiervorrichtung und der Messvorrichtung ermittelten Position eines der Trommelfelle der Person den Tool Center Point, dessen Bewegung im Raum mittels der Steuerungsvorrichtung steuerbar ist, in Relation zu diesem Trommelfell, insbesondere in die Position des relevanten Gleichgewichtsorgans, einzustellen.

Handelt es sich bei der erfindungsgemäßen Halterungsvorrichtung um eine Patientenliege, dann kann die Fixiervorrichtung erste Verschiebevorrichtungen aufweisen, die ein Verschieben der Messvorrichtung in Richtung der Längsachse der Patientenliege erlauben. Die Einstellung der ersten verschiebevorrichtungen erlaubt somit einen Rückschluss auf die Position der Öffnungen der Ohren bezüglich der Längsachse der Patientenliege.

Wenn gemäß einer Variante der erfindungsgemäßen Halterungsvorrichtung in den ersten Verschiebungsvorrichtungen Messvorrichtungen integriert sind, deren Signale ein Maß für die Verschiebung des Messvorrichtungen bezüglich der Längsachse sind, dann ist es in relativ einfacher und automatisierter Weise möglich, die Position der Öffnungen der Ohren bezüglich der Längsachse der Patientenliege zu bestimmen.

Die Fixiervorrichtung kann auch zweite Verschiebevorrichtungen aufweisen, die ein Verschieben der Messvorrichtung in Richtung der Querachse der Patientenliege erlauben. Die Einstellung der zweiten Verschiebevorrichtungen erlaubt somit einen Rückschluss auf die Position der Öffnungen der Ohren bezüglich der Querachse der Patientenliege.

Wenn gemäß einer Ausführungsform der erfindungsgemäßen Halterungsvorrichtung in den zweiten Verschiebungsvorrichtungen Messvorrichtungen integriert sind, deren Signale ein Maß für die Verschiebung des Messvorrichtungen bezüglich der Querachse sind, dann ist es in relativ einfacher und automatisierter Weise möglich, die Position der Öffnungen der Ohren bezüglich der Querachse der Patientenliege zu bestimmen.

Die Fixiervorrichtung kann dritte Verschiebevorrichtungen aufweisen, die ein Verschieben der Messvorrichtung rechtwinklig zu einer Oberfläche der Patientenliege erlauben, auf der die Person liegt. Die Einstellung der dritten Verschiebevorrichtungen erlaubt somit einen Rückschluss auf die Position der Öffnungen der Ohren bezüglich der Oberfläche der Patientenliege.

Wenn gemäß einer Ausführungsform der erfindungsgemäßen Halterungsvorrichtung in den dritten Verschiebungsvorrichtungen Messvorrichtungen integriert sind, deren Signale ein Maß für die Verschiebung des Messvorrichtungen bezüglich der Oberfläche der Patientenliege sind, dann ist es in relativ einfacher und automatisierter Weise möglich, die Position der Öffnungen der Ohren bezüglich der Querachse der Patientenliege zu bestimmen.

Die Fixiervorrichtung kann auch derart ausgestaltet sein, dass diese Gelenkarme aufweist, die z.B. Messskalen und/oder Winkelgeber zum Ermitteln eingestellter Winkel umfasst und den Kopf der Person fixiert. Die Messskalen oder gegebenenfalls die insbesondere mit der Steuerungsvorrichtung verbundenen Messgeber erlauben einen Rückschluss auf die Position der Öffnungen der Gehöhrkanäle der Ohren der Person.

Ein Ausführungsbeispiel der Erfindung ist exemplarisch in den beigefügten schematischen Zeichnungen dargestellt. Es zeigen:
- Fig. 1: einen Roboter mit einer eine Fixiervorrichtung auf- weisenden Patientenliege,
- Fig. 2: eine Seitenansicht der Patientenliege,
- Fig. 3: eine Draufsicht der Patientenliege und
- Fig. 4: ein das Einstellen des Tool Center Points des Robo- ters veranschaulichendes Flussdiagramm.

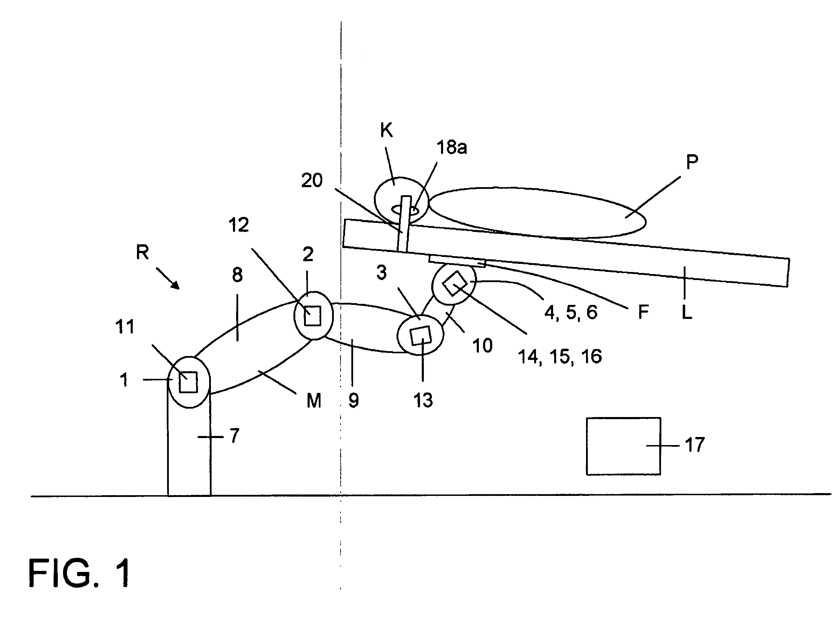

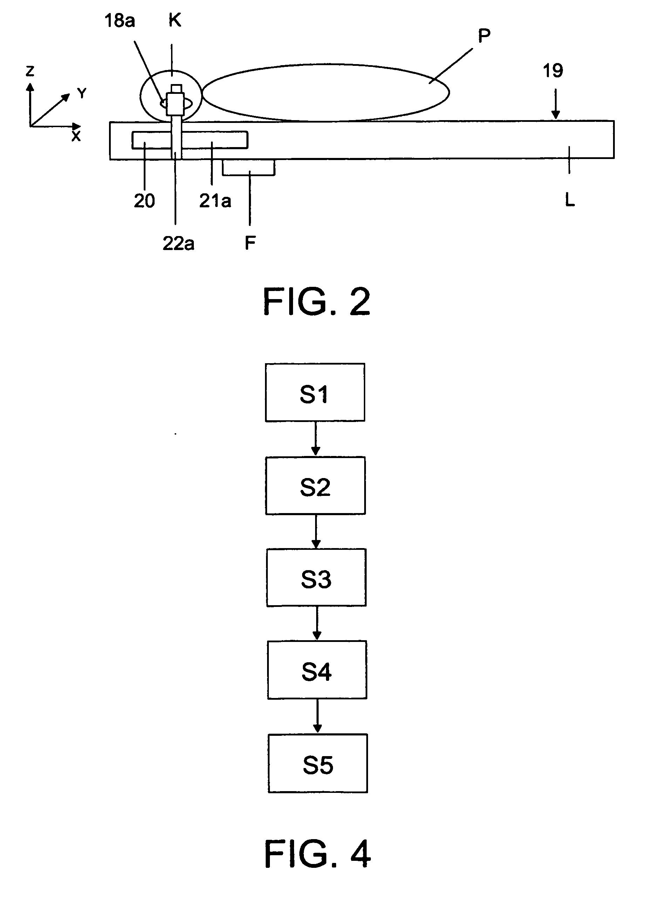

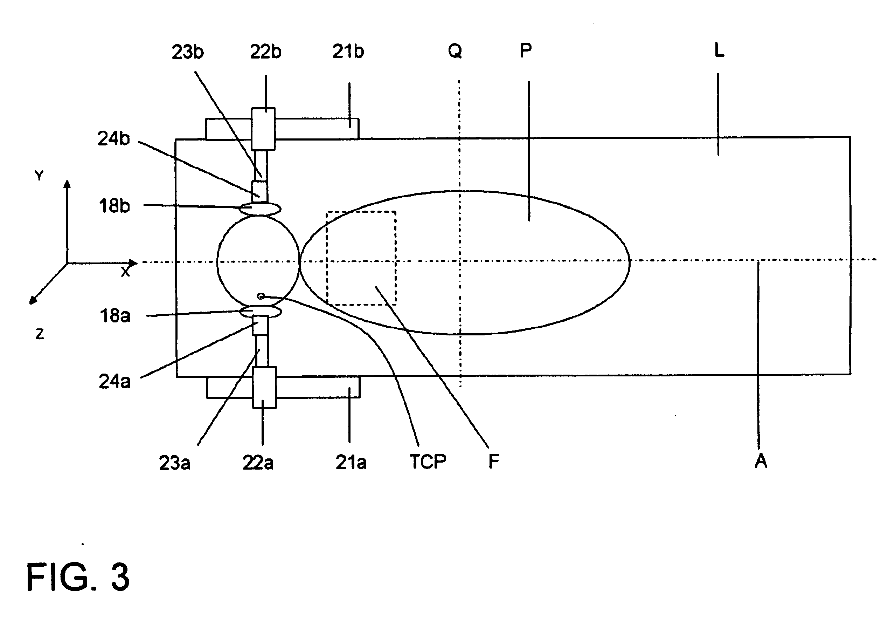

Die Fig. 1 zeigt einen medizinischen Roboter R mit einem Roboterarm M. Der Roboterarm M stellt im Wesentlichen den beweglichen Teil des Roboters R dar und umfasst mehrere Achsen 1-6, mehrere Hebel 7-10 und einen Flansch F, an dem im Falle des vorliegenden Ausführungsbeispiels eine in den Figuren 2 und 3 näher gezeigte Patientenliege L als Beispiel einer Patientenhalterungsvorrichtung befestigt ist. Die Fig. 2 zeigt eine Seitenansicht und die Fig. 3 zeigt eine Draufsicht der Patientenliege L.

Jede der Achsen 1-6 wird mit einem Antrieb, beispielsweise einem elektrischen Antrieb 11-16 bewegt, die in nicht dargestellter Weise mit einem Steuerrechner 17 des Roboters R elektrisch verbunden sind, so dass der Steuerrechner 17 bzw. ein auf dem Steuerrechner 17 laufendes Rechnerprogramm die elektrischen Antriebe 11-16 derart ansteuern kann, dass die Position des Flansches F des Roboters R und somit die Patientenliege L bzw. deren Tool Center Point TCP im Wesentlichen frei im Raum ausgerichtet werden kann. Die elektrischen Antriebe 11-16 des Roboters R umfassen z.B. jeweils einen elektrischen Motor und gegebenenfalls eine die Motoren ansteuernde Leistungselektronik.

Die Patientenliege L weist ferner eine Fixiervorrichtung 20 auf und auf der Patientenliege L liegt eine Person P, deren Kopf K mit der Fixiervorrichtung 20 derart fixiert ist, so dass der Kopf K relativ zur Patientenliege L im Wesentlichen unbeweglich ist.

Im Falle des vorliegenden Ausführungsbeispiels ist der Steuerrechner 17 eingerichtet, den Tool Center Points TCP an die Person P anzugleichen. Die Einstellung des an die Person P angepassten Tool Center Point TCP ist mit einem in der Fig. 4 dargestellten Flussdiagramm zusammengefasst.

Im Falle des vorliegenden Ausführungsbeispiels weist die Fixiervorrichtung 20 in Richtung der Längsachse A (x-Richtung) der Patientenliege L ausgerichtete Verschiebeeinrichtungen in Form von Längsschienen 21a, 21b auf, die jeweils seitlich an der Patientenliege L angebracht sind.

Die Fixiervorrichtung 20 weist im Falle des vorliegenden Ausführungsbeispiels Verschiebevorrichtungen 22a, 22b auf, die in den Längsschienen 21a, 21b verschieblich gelagert sind, so dass die Verschiebvorrichtungen 22a 22b in Richtung der Längsachse A der Patientenliege L verschoben werden können.

Mit den Verschiebevorrichtungen 22a, 22b wirken im Falle des vorliegenden Ausführungsbeispiels bezüglich der zur Längsachse A rechtwinklig ausgerichteten Querachse Q (y-Richtung) teleskopartig ausgeführte Verschiebeeinrichtungen 23a, 23b zusammen, an deren den Seiten der Patientenliege L abgewandten Enden jeweils mit dem Steuerrechner 17 in nicht dargestellter Weise verbundene Messvorrichtungen 24a, 24b befestigt sind. Die teleskopartig ausgeführten Verschiebeeinrichtungen 23a, 23b sind derart mit den Verschiebevorrichtungen 22a, 22b gekoppelt, so dass sie rechtwinklig zur Längsachse A und rechtwinklig zur Querachse Q der Patientenliege L verschoben werden können. Somit ist es möglich, die Messvorrichtungen 24a, 24b in einem gewünschten Abstand zur Oberfläche 19 der Patientenliege L (z-Richtung) auszurichten. Mittels der teleskopartig ausgeführten Verschiebeeinrichtungen 23a, 23b können die Messvorrichtungen 24a, 24b in Richtung der Querachse Q verschoben werden.

Die Fixiervorrichtung 20 ist vorgesehen, den Kopf K der Person P bezüglich der Patientenliege L zu fixieren, indem die Messvorrichtung 24a an das Ohr 18a und die Messvorrichtung 24b an das Ohr 18b der Person P herangeführt werden.

Wenn der Kopf K der Person P fixiert ist, dann kann die Position der Verschiebevorrichtungen 22a, 22b bezüglich der Längsachse A z.B. mittels einer nicht dargestellten, an den Längsschienen 21a, 21b angeordneten Messskalen abgelesen werden. Die Längsschienen 21a, 21b können auch oder alternativ, wie es im vorliegenden Ausführungsbeispiels vorgesehen ist, der Übersicht halber nicht näher dargestellte und mit dem Steuerrechner 17 verbundene Messaufnehmer aufweisen, deren Signale dem Steuerrechner 17 eine Information über die Position der Verschiebevorrichtungen 22a 22b bezüglich der Längsachse A der Patientenliege L und somit der Messvorrichtungen 24a, 24b bezüglich der Längsachse A der Patientenliege L übermitteln.

Die Verschiebevorrichtungen 22a, 22b können ebenfalls eine Messskala umfassen, aufgrund derer die Position der teleskopartig ausgeführten Verschiebeeinrichtungen 23a, 23b relativ zur Oberfläche 19 der Patientenliege L abgelesen werden kann. Die Verschiebevorrichtungen 22a, 22b können auch oder alternativ, wie es im vorliegenden Ausführungsbeispiels vorgesehen ist, der Übersicht halber nicht näher dargestellte und mit dem Steuerrechner 17 verbundene Messaufnehmer aufweisen, deren Signale dem Steuerrechner 17 eine Information über die Position der teleskopartig ausgeführten Verschiebeeinrichtungen 23a 23b bezüglich der Oberfläche 19 der Patientenliege L und somit der Messvorrichtungen 24a, 24b bezüglich der Oberfläche 19 der Patientenliege L übermitteln.

Die teleskopartig ausgeführten Verschiebeeinrichtungen 23a, 23b können ebenfalls eine Messskala umfassen, aufgrund derer die Position der Messvorrichtungen 23a, 23b relativ zur Querachse Q der Patientenliege L abgelesen werden kann. Die Verschiebeeinrichtungen 23a, 23b können auch oder alternativ, wie es im vorliegenden Ausführungsbeispiels vorgesehen ist, der Übersicht halber nicht näher dargestellte und mit dem Steuerrechner 17 verbundene Messaufnehmer aufweisen, deren Signale dem Steuerrechner 17 eine Information über die Position der Messvorrichtungen 24a, 24b bezüglich der Querachse Q der Patientenliege L übermitteln. Somit wird es dem Steuerrechner 17 ermöglicht, die Position der Messvorrichtungen 24a, 24b relativ zur Patientenliege L zu ermitteln, Schritt S1 des in der Fig. 4 gezeigten Flussdiagramms.

Des Weiteren ist die Patientenliege P am Flansch F des Roboters R in einer dem Steuerrechner 17 bekannten Position befestigt und in einer dem Steuerrechner 17 bekannten Orientierung ausgerichtet, so dass der Steuerrechner 17 aufgrund der von den Messaufnehmern stammenden Signalen die Positionen der Messvorrichtungen 24a, 24b relativ zum Flansch F bestimmen kann, Schritt S2 des Flussdiagramms.

Im Falle des vorliegenden Ausführungsbeispiels liegen die Messvorrichtungen 24a, 24b an den Ohren 18a, 18b und insbesondere an den Öffnungen der Ohrkanäle (äußere Gehörgänge). Die Messvorrichtungen 24a, 24b sind im Falle des vorliegenden Ausführungsbeispiels derart ausgeführt, dass sie die Längen der Ohrkanäle, also die Entfernung von den Öffnungen der Ohrkanäle zu den jeweiligen Trommelfellen insbesondere berührungslos ermitteln, Schritt S3 des Flussdiagramms. Berührungslose Messverfahren beruhen z.B. auf Laser oder Ultraschall. Aufgrund der von den Messvorrichtungen 24a, 24b stammenden Signalen ist es dem Steuerrechner 17 demnach möglich, die Positionen der Trommelfelle der Ohren 18a, 18b relativ zum Flansch F des Roboters R zu bestimmen, Schritt S4 des Flussdiagramms.

Im Falle des vorliegenden Ausführungsbeispiels ist der Steuerrechner 17 eingerichtet, den Tool Center Point TCP des Roboters R an die Position eines der Gleichgewichtsorgane (Vestibularorgane) der Ohren 18a, 18b zu legen, Schritt S5 des Flussdiagramms. Aufgrund der Position des relevanten Trommelfells wird es dem Steuerrechner 17 demnach ermöglicht, auf die Position des relevanten Gleichgewichtorgans relativ zur Patientenliege L und somit relativ zum Flansch F zu schließen, um den Tool Center Point TCP des Roboters R an die Position des relevanten Gleichgewichtorgans zu setzt.

Im Falle des vorliegenden Ausführungsbeispiels ist der Steuerrechner 17 derart konfiguriert, die Antrieb 11-16 derart anzusteuern, so dass der Tool Center Point TCP, und somit die auf der Patientenliege L liegende Person P eine vorbestimmte Bewegung durchführt.

Anstelle der Verschiebeeinrichtungen in Form von Längsschienen 21a, 21b und der Verschiebeeinrichtungen 23a, 23b kann die Fixiervorrichtung 20 auch anderweitig konstruktiv ausgeführt sein. Eine alternative Ausprägung der Fixiervorrichtung 20 kann auch derart sein, dass diese Gelenkarme aufweist, die z.B. Messskalen und/oder Winkelgeber zum Ermitteln eingestellter Winkel umfasst und den Kopf K der Person P fixiert. Die Messskalen bzw. die insbesondere mit dem Steuerrechner 17 verbundenen Messgeber erlauben einen Rückschluss auf die Position der Öffnungen der Gehöhrkanäle der Ohren (18a, 18b), so dass der Roboter R die Achsen 1-6 gegebenenfalls geeignet ansteuern kann.

## Patentansprüche

1. Halterungsvorrichtung zum Halten einer Person (P), aufweisend eine Fixiervorrichtung (20) zum Fixieren des Kopfs (K) einer von der Halterungsvorrichtung (L) gehaltenen Person (P), wobei die Fixiervorrichtung (20) derart ausgeführt ist, dass sie aufgrund ihrer dem Kopf (K) der Person (P) zugeordneten Einstellung einen Rückschluss auf die Position der Öffnungen der Gehörkanäle der Ohren (18a, 18b) der Person (P) relativ zur Halterungsvorrichtung (20) erlaubt, **dadurch gekennzeichnet, dass** die Fixiervorrichtung (20) Messvorrichtungen aufweist, deren Signale einen Rückschluss auf die Position der Öffnungen der Gehörkanäle der Ohren (18a, 18b) der Person (P) erlauben.

2. Halterungsvorrichtung nach Anspruch 1, deren Fixiervorrichtung (20) eine Messvorrichtung (24a, 24b) aufweist, die bei der dem Kopf (K) der Person (P) zugeordneten Einstellung an den Öffnungen der Gehörkanälen anliegt und die Entfernung zu den Trommelfellen der Person (P) ermittelt.

3. Haltevorrichtung nach Anspruch 1 oder 2, die als Patientenliege (L) ausgebildet ist.

4. Halterungsvorrichtung nach Anspruch 3, deren Fixiervorrichtung (20) erste Verschiebevorrichtungen (21a, 21b) aufweist, die ein Verschieben der Messvorrichtung (24a, 24b) in Richtung der Längsachse (A) der Patientenliege (L) erlauben.

5. Haltevorrichtung nach Anspruch 4, bei der in die ersten Verschiebungsvorrichtungen (21a, 21b) Messvorrichtungen integriert sind, deren Signale ein Maß für die Verschiebung des Messvorrichtungen (24a, 24b) bezüglich der Längsachse (A) sind.

6. Halterungsvorrichtung nach einem der Ansprüche 3 bis 5, deren Fixiervorrichtung (20) zweite Verschiebevorrichtungen (23a, 23b) aufweist, die ein Verschieben der Messvorrichtung (24a, 24b) in Richtung der Querachse (Q) der Patientenliege (L) erlauben.

7. Haltevorrichtung nach Anspruch 6, bei der in die zweiten Verschiebungsvorrichtungen (23a, 23b) Messvorrichtungen integriert sind, deren Signale ein Maß für die Verschiebung des Messvorrichtungen (24a, 24b) bezüglich der Querachse (Q) sind.

8. Halterungsvorrichtung nach einem der Ansprüche 3 bis 7, deren Fixiervorrichtung (20) dritte Verschiebevorrichtungen (22a, 22b) aufweist, die ein Verschieben der Messvorrichtung (24a, 24b) rechtwinklig zu einer Oberfläche (19) der Patientenliege (L) erlauben, auf der die Person (P) liegt.

9. Haltevorrichtung nach Anspruch 8, bei der in die dritten Verschiebungsvorrichtungen (22a, 22b) Messvorrichtungen integriert sind, deren Signale ein Maß für die Verschiebung des Messvorrichtungen (24a, 24b) rechtwinklig zur Oberfläche (19) der Patientenliege (L) sind.

10. Halterungsvorrichtung nach einem der Ansprüche 1 bis 3, deren Fixiervorrichtung (20) insbesondere an der Patientenliege (L) angeordnete Gelenkarme aufweist, die insbesondere Messskalen und/oder Winkelgeber zum Ermitteln eingestellter Winkel umfasst, die einen Rückschluss auf die Position der Öffnungen der Gehörkanäle der Ohren (18a, 18b) der Person (P) relativ zur Haltevorrichtung (20) erlauben.

11. Medizinischer Roboter, aufweisend
- eine Halterungsvorrichtung (L) nach einem der Ansprüche 1 bis 10,
- eine Steuerungsvorrichtung (17) und
- einen Roboterarm (M), der von der Steuerungsvorrichtung (17) angesteuerte mehrere Achsen (1-6) und eine Befestigungsvorrichtung (F) aufweist, wobei die Halterungsvorrichtung (L) an der Befestigungsvorrichtung (F) befestigt ist.

12. Roboter nach Anspruch 11, der eingerichtet ist, aufgrund der mittels der Fixiervorrichtung (20) ermittelten Position einer der Öffnungen der Gehörkanäle der Person (P) den Tool Center Point (TCP), dessen Bewegung im Raum mittels der Steuerungsvorrichtung (17) steuerbar ist, in Relation zu dieser Öffnung, insbesondere in die Position des relevanten Gleichgewichtsorgans einzustellen.

13. Roboter nach Anspruch 11, der eingerichtet ist, aufgrund der mittels der Fixiervorrichtung (20) und der Messvorrichtung (24a, 24b) ermittelten Position eines der Trommelfelle der Person (P) den Tool Center Point (TCP), dessen Bewegung im Raum mittels der Steuerungsvorrichtung (17) steuerbar ist, in Relation zu diesem Trommelfell, insbesondere in die Position des relevanten Gleichgewichtsorgans, einzustellen.

14. Verfahren zum Einstellen des Tool Center Points (TCP) eines Roboters (R), der einen Roboterarm (M) mit einer Befestigungsvorrichtung (F) aufweist, an der eine Halterungsvorrichtung (L) mit einer Fixiervorrichtung (20) insbesondere nach einem der Ansprüche 1 bis 10 befestigt ist, aufweisend folgende Verfahrensschritte:
- Fixieren des Kopfes (K) einer mit der Halterungsvorrichtung (20) gehaltenen Person (P) mittels der Fixiervorrichtung (20),
- Ermitteln der Position eines der beiden Trommelfelle der Person (P) mittels Messvorrichtungen (24a, 24b) der Fixiervorrichtung (2) relativ zur Halterungsvorrichtung (L),
- Ermitteln der Position des dem Trommelfell zugeordneten Gehörorgans relativ zur Halterungsvorrichtung (L) und
- Legen des Tool Center Points (TCP) des Roboters (R) in die Position des Gehörorgans.

## Claims

1. A holding device for holding a person (P) comprising a fixation device (20) for fixing the head (K) of the person (P) being held in the holding device (L), wherein the fixation device (20) is configured such that it permits the determination of the position of the openings of the ear canals of the ears (18a, 18b) of the person (P) relative to the holding device (20) due to its setting based on the head (K) of the person (P), **characterized in that** the fixation device (20) comprises measuring devices whose signals enable the conclusion of the position of the openings of the ear canals of the ears (18a, 18b) of the person (P)

2. Holding device according to claim 1, whose fixation device (20) comprises a measuring device (24a, 24b) resting on the head (K) of the person (P) set up according to the openings of the ear canals and which determines the distance to the eardrums of the person (P).

3. Holding device according to claim 1 or 2, which is designed as a patient bed (L).

4. Holding device according to claim 3, whose fixation device (20) comprises first displacement devices (21a, 21b) which allow shifting of the measuring device (24a, 24b) towards the longitudinal axis (A) of the patient bed (L).

5. Holding device according to claim 4, wherein measuring devices are integrated in the first displacement devices (21a, 21b), whose signals are a measure of the displacement of the measuring devices (24a, 24b) relative to the longitudinal axis (A).

6. Holding device according to one of the claims 3 to 5, whose fixation device (20) comprises second displacement devices (23a, 23b) which allow shifting of the measuring device (24a, 24b) towards the transverse axis (Q) of the patient bed (L).

7. Holding device according to claim 6, in which measurement devices are integrated in the second displacement measurement devices (23a, 23b), whose signals are a measure of the displacement of the measuring devices (24a, 24b) relative to the transverse axis (Q).

8. Holding device according to one of the claims 3 to 7, whose fixation device (20) comprises third displacement devices (22a, 22b), which permit a perpendicular displacement of the measurement device (24a, 24b) to a surface (19) of the patient bed (L), on which the person (P) lies.

9. Holding device according to claim 8, in which measurement devices are integrated in the third displacement measurement devices (22a, 22b), whose signals are a measure of the displacement of the measuring devices (24a, 24b) perpendicular to the surface (19) of the patient bed (L).

10. Holding device according to one of the claims 1 to 3, whose fixation device (20) comprises articulated arms particularly arranged at the patient bed (L) which comprise in particular measurement scales and / or angle sensors to determine the set angle, which permit the determination of the position of the openings of the ear canals of the ears (18a, 18b) of the person (P) relative to the holding device (20).

11. Medical robot comprising
- a holding device (L) according to one of the claims 1 to 10,
- a control device (17) and
- a robot arm (M) comprising several axes (1-6) controlled by the control device (17) and a fastening device (F), wherein the holding device (L) is attached to the fastening device (F).

12. Robot according to claim 11, which is configured by means of the position determined by the holding device (20) of the opening of one of the ear canals of the person (P) to adjust the Tool Center Point (TCP), whose movement in space is controlled by means of the control device (17) relative to this opening, in particular in the position of the relevant organ of equilibrium.

13. Robot according to claim 11, which is configured by means of the position determined by the fixation device (20) and the measuring device (24a, 24b) of one of the ear drums of the person (P) to adjust the Tool Center Point (TCP), whose movement in space is controlled by means of the control device (17) relative to the ear drum, in particular in the position of the relevant organ of equilibrium.

14. A method for setting the Tool Center Point (TCP) of a robot (R), which comprises a robot arm (M) with a fastening device (F), in which a holding device (L) with a fixation device (20), particularly according to one of claims 1 to 10 is attached, comprising the following steps:
- fixing of the head (K) of a person (P) held by the fixation device (20),
- determining the position of one of the two eardrums of the person (P) by means of measuring devices (24a, 24b) of the holding device (20) relative to holding device (L),
- determining the position of the organ of hearing associated with the ear drum relative to the holding device (L) and
- inserting the Tool Center Point (TCP) of the robot (R) in the position of the organ of hearing.

## Revendications

1. Dispositif de retenue pour maintenir une personne (P), présentant un dispositif d'immobilisation (20) pour immobiliser la tête (K) d'une personne (P) maintenue par le dispositif de retenue (L), le dispositif d'immobilisation (20) étant réalisé de telle sorte qu'en raison de son réglage associé à la tête (K) de la personne (P), il permet de faire une déduction sur la position des orifices des conduits auditifs des oreilles (18a, 18b) de la personne (P) par rapport au dispositif de retenue (20), **caractérisé en ce que** le dispositif d'immobilisation (20) présente des dispositifs de mesure dont les signaux permettent de faire une déduction sur la position des orifices des conduits auditifs des oreilles (18a, 18b) de la personne (P).

2. Dispositif de retenue selon la revendication 1, dont le dispositif d'immobilisation (20) présente un dispositif de mesure (24a, 24b) qui, pour le réglage associé à la tête (K) de la personne (P), est en appui sur les orifices des conduits auditifs et détermine la distance par rapport aux tympans de la personne (P).

3. Dispositif de retenue selon la revendication 1 ou 2, qui est réalisé sous forme de table de patient (L).

4. Dispositif de retenue selon la revendication 3, dont le dispositif d'immobilisation (20) présente des premiers dispositifs de déplacement (21a, 21b) qui permettent un déplacement du dispositif de mesure (24a, 24b) en direction de l'axe longitudinal (A) de la table de patient (L).

5. Dispositif de retenue selon la revendication 4, dans lequel dans les premiers dispositifs de déplacement (21a, 21b) sont intégrés des dispositifs de mesure dont les signaux sont une grandeur pour le déplacement des dispositifs de mesure (24a, 24b) par rapport à l'axe longitudinal (A).

6. Dispositif de retenue selon l'une des revendications 3 à 5, dont le dispositif d'immobilisation (20) présente deux dispositifs de déplacement (23a, 23b) qui permettent un déplacement des dispositifs de mesure (24a, 24b) en direction de l'axe transversal (Q) de la table de patient (L).

7. Dispositif de retenue selon la revendication 6, dans lequel dans les deuxièmes dispositifs de déplacement (23a, 23b) sont intégrés des dispositifs de mesure dont les signaux sont une grandeur pour le déplacement des dispositifs de mesure (24a, 24b) par rapport à l'axe transversal (Q).

8. Dispositif de retenue selon l'une des revendications 3 à 7, dont le dispositif d'immobilisation (20) présente des troisièmes dispositifs de déplacement (22a, 22b) qui permettent un déplacement du dispositif de mesure (24a, 24b) perpendiculairement à une surface (19) de la table de patient (L) sur laquelle est allongée la personne (P).

9. Dispositif de retenue selon la revendication 8, dans lequel dans les troisièmes dispositifs de déplacement (22a, 22b) sont intégrés des dispositifs de mesure dont les signaux sont une grandeur pour le déplacement des dispositifs de mesure (24a, 24b) perpendiculairement à la surface (19) de la table de patient (L).

10. Dispositif de retenue selon l'une des revendications 1 à 3, dont le dispositif d'immobilisation (20) présente en particulier des bras articulés agencés sur la table de patient (L), qui comprennent en particulier des échelles de mesure et/ou des capteurs angulaires pour déterminer des réglages d'angles qui permettent de faire une déduction sur la position des orifices des conduits auditifs des oreilles (18a, 18b) de la personne (P) par rapport au dispositif de retenue (20).

11. Robot médical comprenant :
- un dispositif de retenue (L) selon l'une des revendications 1 à 10,
- un dispositif de commande (17) et
- un bras de robot (M) qui présente plusieurs axes (1 à 6) pilotés par le dispositif de commande (17) et un dispositif de fixation (F), le dispositif de retenue (L) étant fixé sur le dispositif de fixation (F).

12. Robot selon la revendication 11, qui est aménagé pour régler, sur la base de la position d'un des orifices des conduits auditifs de la personne (P), laquelle est déterminée au moyen du dispositif d'immobilisation (20), le point central virtuel d'outil (TCP), dont le mouvement dans l'espace peut être commandé par le dispositif de commande (17), en relation avec cet orifice, en particulier dans la position de l'organe équilibreur concerné.

13. Robot selon la revendication 11, qui est aménagé pour régler, sur la base de la position d'un des tympans de la personne (P), laquelle est déterminée au moyen du dispositif d'immobilisation (20) et du dispositif de mesure (24a, 24b), le point central virtuel d'outil (TCP) dont le mouvement dans l'espace peut être commandé par le dispositif de commande (17), en relation avec ce tympan, en particulier dans la position de l'organe équilibreur concerné.

14. Procédé de réglage du point central virtuel d'outil (TCP) d'un robot (R) qui présente un bras de robot (M) avec un dispositif de fixation (F) sur lequel est fixé un dispositif de retenue (L) avec un dispositif d'immobilisation (20) en particulier selon l'une des revendications 1 à 10, présentant les étapes de procédé suivantes :
- immobilisation de la tête (K) d'une personne (P) maintenue avec le dispositif de retenue (L) au moyen du dispositif d'immobilisation (20),
- détermination de la position d'un des deux tympans de la personne (P) au moyen de dispositifs de mesure (24a, 24b) du dispositif d'immobilisation (20) par rapport au dispositif de retenue (L),
- détermination de la position du l'organe de l'ouïe associé au tympan, par rapport au dispositif de retenue (L), et
- mise en place du point central virtuel d'outil (TCP) du robot (R) dans la position de l'organe auditif.
